# EUROPÄISCHE PATENTSCHRIFT

(11) **EP 1 506 319 B1**
(45) Veröffentlichungstag und Bekanntmachung des Hinweises auf die Patenterteilung: **05.08.2009**
(21) Anmeldenummer: 03749808.6
(22) Anmeldetag: 02.05.2003
(51) Int. Cl.: C14C 3/16, C07C 45/72

(54) **GERBMITTEL UND KONSERVIERUNGSMITTEL AUF DER BASIS VON DIALDEHYDEN**
TANNING AGENT AND CURING AGENT BASED ON DIALDEHYDES
MATIERES TANNANTES ET AGENTS DE CONSERVATION A BASE DE DIALDEHYDES

(30) Priorität: 07.05.2002 DE 10220493; 10.07.2002 DE 10231293
(43) Veröffentlichungstag der Anmeldung: 16.02.2005
(73) Patentinhaber: BASF SE, 67056 Ludwigshafen (DE)
(72) Erfinder: HÜFFER, Stephan, 67063 Ludwigshafen (DE); PABST, Gunther, 92318 Neumarkt (DE); TAEGER, Tilman, Lüdecke, 64342 Seeheim-Jugenheim (DE); SCHROEDER, Stefan, 67271 Neuleiningen (DE); WOLF, Gerhard, 68775 Ketsch (DE)
(86) Internationale Anmeldenummer: PCT/EP2003/004603
(87) Internationale Veröffentlichungsnummer: WO 2003/095681

(56) Entgegenhaltungen:
- DE-A- 2 003 600
- DE-A- 2 215 948
- DE-A- 4 444 709
- DE-C- 3 811 267

## Beschreibung

Die vorliegende Erfindung betrifft Gerbmittel, herstellbar durch Umsetzen mindestens eines Aldehyds der allgemeinen Formel I, 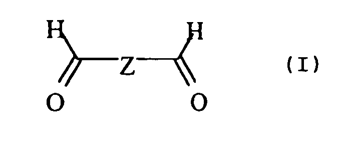 mit mindestens einem weiteren identischen oder verschiedenen Aldehyd der Formel I,
wobei die Variablen wie folgt definiert sind:
- Z: eine chemische Einfachbindung, gegebenenfalls substituiertes C₁-C₁₂-Alkylen, gegebenenfalls substituiertes C₅-C₁₂-Cycloalkylen oder gegebenenfalls substituiertes C₆-C₁₄-Arylen,
wobei man die Umsetzung in Anwesenheit eines sauren Katalysators und in Gegenwart mindestens einer weiteren Carbonylverbindung der Formel II 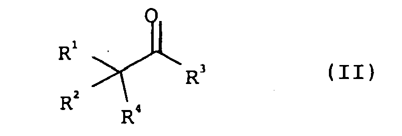 durchführt,
wobei R¹ bis R⁴ unabhängig voneinander Wasserstoff, gegebenenfalls substituiertes C₁-C₁₂-Alkyl, gegebenenfalls substituiertes C₁-C₁₂-Cycloalkyl, gegebenenfalls substituiertes C₇-C₁₃-Aralkyl oder gegebenenfalls substituiertes C₆-C₁₄-Aryl bedeuten,
mit der Maßgabe, dass für den Fall, dass Z einer chemischen Einfachbindung oder einem Rest ohne α-Wasserstoffatomen entspricht, mindestens ein weiterer Aldehyd der Formel I, in welchem der Rest Z α-Wasserstoffatome enthält, oder mindestens eine weitere Carbonylverbindung der Formel II, die α H-Atome trägt, vorhanden ist.

Desweiteren betrifft die vorliegende Erfindung ein Verfahren zur Herstellung dieser Gerbmittel, ihre Verwendung als Konservierungsmittel, insbesondere ihre Verwendung zum Vorgerben, Gerben oder Nachgerben von Tierhäuten, sowie ein Verfahren zum Vorgerben, Gerben oder Nachgerben von Tierhäuten mittels der erfindungsgemäßen Gerbmittel.

Weiter betrifft die vorliegende Erfindung einen pulverförmigen Wirkstoff, welcher ein oder mehrere der erfindungsgemäßen Gerbmittel enthält, ein Verfahren zur Herstellung dieses pulverförmigen Wirkstoffs und seine Verwendung als Konservierungsmittel sowie Leder, welches unter Verwendung der erfindungsgemäßen Gerbmittel oder nach dem erfindungsgemäßen Verfahren hergestellt worden ist.

Seit über 100 Jahren ist die Chromgerbung in der Lederherstellung eine wichtige chemische Behandlung, siehe beispielsweise Ullmann's Encyclopedia of Industrial Chemistry, Band A15, Seite 259 bis 282 und insbesondere Seite 268 ff., 5. Auflage, (1990), Verlag Chemie Weinheim). Aus ökologischen Gründen wird jedoch nach
Alternativen zur Chromgerbung gesucht. Bei der herkömmlichen Chromgerbung werden Chromsalze in einer Menge von 1,5 bis 8 Gew.-%, bezogen auf das Blößengewicht des Leders, oder sogar mehr angeboten. Hiervon wird meist ein erheblicher Teil nicht gebunden und gelangt ins Abwasser. Zwar gelingt es, das Abwasser durch chemische Behandlung mit beispielsweise Kalk und Eisensalzen von beträchtlichen Anteilen Chrom zu befreien, es fallen jedoch Chrom-haltige Schlämme an, die auf Sonderdeponien entsorgt oder aufwändig aufgearbeitet werden müssen.

Außerdem fallen beispielsweise beim Spalten der Häute und beim Egalisieren der Leder Chrom-haltige Lederabfälle an, die etwa 8 bis 15 Gew.-%, bezogen auf das Hautgewicht, ausmachen können und ebenfalls aufwändig entsorgt werden müssen.

Es hat nicht an Versuchen gefehlt, durch beispielsweise Recyclieren der Chromgerbflotten oder Chromrecyclingverfahren die Chrombelastung der Abwässer zu verringern. Diese Verfahren waren aber im Ganzen unbefriedigend und insbesondere nicht in der Lage, das Problem der Chrom-haltigen Lederabfälle zu lösen.

Bekannt sind des weiteren Verfahren, in denen das Chrom ganz oder teilweise durch organische Gerbmittel ersetzt wurden. Genannt seien der Einsatz der sogenannten Syntane, das sind sulfonierte Kondensationsprodukte von Formaldehyd und Phenol oder sulfonierte Naphthalin-Formaldehyd-Kondensationsprodukte. Genannt sei weiterhin der Einsatz sogenannter vegetabiler Gerbstoffe. Beide Klassen von Gerbmitteln sorgen jedoch für einen erhöhten CSB-Wert der Abwässer und sind aus Umweltgründen ebenfalls nicht unbedenklich. Außerdem hat sich erwiesen, dass die Lichtechtheit der Leder bei der Verwendung von sulfonierten Phenol/Formaldehyd-Kondensationsprodukten oft zu wünschen übrig lässt *(*Ullmann's Encyclopedia of Industrial Chemistry, Band A15, Seite 259 bis 282 und insbesondere Seite 270 ff., 5. Auflage, (1990), Verlag Chemie Weinheim).

Bekannt ist weiterhin die Verwendung von Polymergerbstoffen, beispielsweise aus EP-A 0 792 377. Die anwendungstechnischen Eigenschaften der nach dem offenbarten Verfahren erhaltenen Leder lassen sich jedoch noch verbessern.

Weiterhin ist die Gerbung unter Verwendung von Aldehyden, insbesondere Dialdehyden wie beispielsweise Glutardialdehyd, bekannt, siehe beispielsweise H. Herfeld, Bibliothek des Leders, Band III, Seite 191, Umschau Verlag Frankfurt/Main, 1984. Nachteilig ist jedoch, dass bei geringen Mengen an Glutardialdehyd, beispielsweise 0,5 bis 0,9 Gew.-% bezogen auf das Blößengewicht), die Schrumpfungstemperaturen nicht über 70°C hinausgehen und dass sich die erzeugten Halbfabrikate daher nur unzureichend entwässern lassen. Während des Falzens tritt eine Verleimung auf der Fleischseite des Leders auf und beeinflusst die Qualität des Leders nachteilig. Andererseits wird man aber unter arbeitshygienischen Gesichtspunkten die Verwendung von größeren Mengen an Glutardialdehyd zu vermeiden versuchen.

Es ist auch bekannt, Glutardialdehyd in partiell oder ganz acetalisierter Form zum Gerben einzusetzen, beispielsweise als Methylacetal (Ullmann's Encyclopedia of Industrial Chemistry, Band A15, Seite 259 bis 282 und insbesondere Seite 273 ff., 5. Auflage, (1990), Verlag Chemie Weinheim). Die beschriebenen gegerbten Halbfabrikate neigen jedoch im Allgemeinen rasch zum Vergilben.

In DE-A 20 03 600 werden durch basische Katalyse hergestellte Aldolisierungsprodukte in hoher Reinheit aus Isobutyraldehyd und Glyoxal offenbart, die zum Gerben und zur Konservierung verwendet werden.

Aus DE-A 22 15 948 ist ein Gerbstoff, der aus Glutardialdehyd und Formaldehyd mittels Sodalösung hergestellt wird, bekannt. Damit gegerbtes Leder zeigt veringerte Vergilbung.

In DE-C 38 11 267 wird offenbart, dass eine Acetalisierung von Glutardialdehyd oder anderen Dialdehyden, 2 bis 8 C-Atome haben, mit kurzkettigen Alkylglykolen, Alkylpolyglykolen, aliphatischen Alkoholen, Glycerin oder Sacchariden vorteilhafte Effekte bringt. Jedoch ist der Dampfdruck der Dialdehyde, die leicht aus den sehr hydrolyseempfindlichen Acetalen rückgebildet wird, noch immer merklich. Auch lassen sich die anwendungstechnischen Eigenschaften der so erhaltenen Leder weiter verbessern.

Es bestand daher die Aufgabe, neue Gerbmittel für die Vorgerbung, Gerbung und Nachgerbung von Tierhäuten bereit zu stellen, das die oben genannten Nachteile vermeidet. Insbesondere bestand die Aufgabe, ein Gerbmittel bereit zu stellen, das die oben beschriebenen Nachteile vermeidet.

Demgemäß wurden die eingangs definierten Gerbmittel gefunden.

### In Formel I

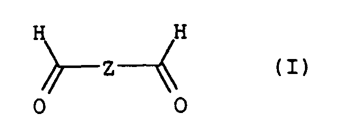

sind die Variablen wie folgt definiert:
Z eine chemische Einfachbindung,
   gegebenenfalls substituiertes C₁-C₁₂-Alkylen, wie beispielsweise -CH₂-, -CH₂-CH₂-, - (CH₂) ₃-, - (CH₂) ₄-, - (CH₂) ₅-, (CH₂) ₆-, -(CH₂)₇-, -(CH₂)₈-, -(CH₂)₉-, - (CH₂)₁₀-, -(CH₂)₁₁-, -(CH₂)₁₂-, cis- oder trans-CH=CH-, Z- oder E-CH₂-CH=CH-; bevorzugt -CH₂-, -CH₂-CH₂-, -(CH₂)₃-, -(CH₂)₄-, -CH(CH₃)-, -CH(C₆H₅)-, -CH(CH₃)-CH₂-, syn-CH(CH₃)-CH(CH₃)-, anti-CH(CH₃)-CH(CH₃)-, - syn-CH(CH₃)-CH(C₆H₅)-, anti-CH(CH₃)-CH(C₆H₆)-, -{CH(CH₃)}₃-,
   gegebenenfalls substituiertes C₅-C₁₂-Cycloalkylen, wie beispielsweise trans- oder cis-1,2-Cyclopentanylen, trans- oder cis-1,3-Cyclopentanylen, trans- oder cis-1,3-Cyclopent-4-eny-len, trans- oder cis-1,4-Cyclohexanylen, trans- oder cis-1,4-Cyclohex-2-enylen, trans- oder cis-1,3-Cyclohexanylen, trans-oder cis-1,2-Cyclohexanylen, gegebenenfalls substituiert mit einem oder mehreren C₁-C₄-Alkylgruppen, wie beispielsweise Methyl, Ethyl, n-Propyl, iso-Propyl, n-Butyl, sek.-Butyl, iso-Butyl oder tert.-Butyl, oder einem oder mehreren Halogenatomen, wie beispielsweise Fluor, Chlor, Brom oder Iod, oder
   gegebenenfalls substituiertes C₆-C₁₄-Arylen, wie beispielsweise para-Phenylen, meta-Phenylen, ortho-Phenylen, 1,2-Naphthylen, 1,3-Naphthylen, 1,4-Naphthylen, 1,5-Naphthylen, 1,6-Naphthylen, 1,7-Naphthylen, 1,8-Naphthylen 2,3-Naphthylen, 2,7-Naphthylen, 2,6-Naphthylen, 1,4-Anthrylen, 9,10-Anthrylen, p,p'-Biphenylen, gegebenenfalls substituiert mit einem oder mehreren C₁-C₄-Alkylgruppen, wie beispielsweise Methyl, Ethyl, n-Propyl, iso-Propyl, n-Butyl, sek.-Butyl, iso-Butyl oder tert.-Butyl, oder einem oder mehreren Halogenatomen, wie beispielsweise Fluor, Chlor, Brom oder Iod.
   In Formel II 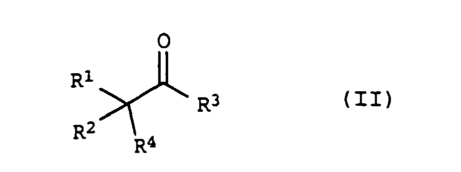 bedeuten:
R¹ bis R⁴ unabhängig voneinander Wasserstoff,
   gegebenenfalls substituiertes C₁-C₁₂-Alkyl, wie etwa Methyl, Ethyl, n-Propyl, iso-Propyl, n-Butyl, iso-Butyl,- sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl und ganz besonders bevorzugt Methyl;
   gegebenenfalls substituiertes C₃-C₁₂-Cycloalkyl, wie etwa Cyclopropyl, Cyclobutyl, Cyclopentyl, Cyclohexyl, Cycloheptyl, Cyclooctyl, Cyclononyl, Cyclodecyl, Cycloundecyl und Cyclododecyl; bevorzugt sind Cyclopentyl, Cyclohexyl, Cycloheptyl, 2-Methylcyclopentyl, 3-Methylcyclopentyl, cis-2,4-Dimethylcy-clopentyl, trans-2,4-Dimethylcyclopentyl 2,2,4,4-Tetramethylcyclopentyl, 2-Methylcyclohexyl, 3-Methylcyclohexyl, 4-Methylcyclohexyl, cis-2,5-Dimethylcyclohexyl, trans-2,5-Dimethylcyclohexyl, 2,2,5,5-Tetramethylcyclohexyl, 2-Methoxycyclopentyl, 2-Methoxycyclohexyl, 3-Methoxycyclopentyl, 3-Methoxycyclohexyl, 2-Chlorcyclopentyl, 3-Chlorcyclopentyl, 2,4-Dichlorcyclopentyl, 2,2,4,4-Tetrachlorcyclopentyl, 2-Chlorcyclohexyl, 3-Chlorcyclohexyl, 4-Chlorcyclohexyl, 2,5-Dichlorcyclohexyl, 2,2,5,5-Tetrachlorcyclohexyl, 2-Thiomethylcyclopentyl, 2-Thiomethylcyclohexyl, 3-Thiomethyl-cyclopentyl, 3-Thiomethylcyclohexyl und weitere Derivate;
   gegebenenfalls substituiertes C₇-C₁₃-Aralkyl, bevorzugt C₇-bis C₁₂-Phenylalkyl, wie etwa Benzyl, 1-Phenethyl, 2-Phenethyl, 1-Phenyl-propyl, 2-Phenyl-propyl, 3-Phenyl-propyl, Neophyl (1-Methyl-1-phenylethyl), 1-Phenyl-butyl, 2-Phenyl-butyl, 3-Phenyl-butyl und 4-Phenyl-butyl, besonders bevorzugt Benzyl, oder
   gegebenenfalls substituiertes C₆-C₁₄-Aryl, wie beispielsweise Phenyl, 1-Naphthyl, 2-Naphthyl, 1-Anthryl, 2-Anthryl, 9-Anthryl, 1-Phenanthryl, 2-Phenanthryl, 3-Phenanthryl, 4-Phenanthryl und 9-Phenanthryl, bevorzugt Phenyl, 1-Naphthyl und 2-Naphthyl, besonders bevorzugt Phenyl, wobei als Substituenten der C₆-C₁₄-Arylgruppen in Betracht kommen:
   C₁-C₁₂-Alkylgruppen, wie etwa Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, n-Heptyl, iso-Heptyl, n-Octyl, n-Nonyl, n-Decyl, und n-Dodecyl; bevorzugt C₁-C₆-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, iso-Amyl, n-Hexyl, iso-Hexyl, sec.-Hexyl, besonders bevorzugt C₁-C₄-Alkyl wie Methyl, Ethyl, n-Propyl, iso-Propyl, n-Butyl, iso-Butyl, sec.-Butyl und tert.-Butyl;
   Halogene, wie etwa Fluor, Chlor, Brom und Jod, wobei Chlor und Brom bevorzugt sind, und/oder
   C₁-C₁₂-Alkoxygruppen, bevorzugt C₁-C₆-Alkoxygruppen, wie etwa Methoxy, Ethoxy, n-Propoxy, iso-Propoxy, n-Butoxy, iso-Butoxy, sec.-Butoxy, tert.-Butoxy, n-Pentoxy, iso-Pentoxy, n-Hexoxy und iso-Hexoxy, besonders bevorzugt Methoxy, Ethoxy, n-Propoxy und n-Butoxy.
In einer besonderen Ausführungsform sind die Reste R¹ und R² in Formel II miteinander unter Bildung eines 4- bis 13-gliedrigen Rings kovalent verbunden. So können R¹ und R² beispielsweise gemeinsam sein: -(CH₂)₄-, -(CH₂)₅-, -(CH₂)₆, -(CH₂)₇-, -CH(CH₃)-CH₂-CH₂-CH(CH₃)- oder -CH(CH₃)-CH₂-CH₂-CH₂-CH(CH₃)-.

Bevorzugt sind R¹ und R² jeweils Wasserstoff.

Ganz besonders bevorzugt ist Z gleich -(CH₂)₃-.

Die erfindungsgemäße Umsetzung erfolgt vorzugsweise durch Erhitzen auf Temperaturen von 30 bis 130°C, insbesondere 20 bis 100°C und ganz besonders bei 50 bis 80°C. Man kann die Umsetzung bei beliebigen Drücken von 0,1 bis 100 bar durchführen, bevorzugt ist Atmosphärendruck. Die Umsetzung kann in Gegenwart eines Lösemittels erfolgen, beispielsweise von Kohlenwasserstoffen wie vorzugsweise Toluol, Petrolether oder n-Heptan. Auch halogenierte Kohlenwasserstoffe wie beispielsweise Chloroform sind grundsätz-. lich geeignet. Bevorzugt ist die Umsetzung in wässriger Lösung oder wässriger Dispersion.

Man kann zur Beschleunigung der Reaktion wasserentziehende Mittel zusetzen, aber der Zusatz von wasserentziehender Mittel ist nicht notwendig. Führt man die Umsetzung in Wasser als Lösemittel durch, so ist der Zusatz von wasserentziehenden Mitteln natürlich nicht sinnvoll.

Als Katalysator werden erfindungsgemäß saure Katalysatoren eingesetzt, beispielsweise -Phosphorsäure, Ameisensäure, Essigsäure, saure Kieselgele, verdünnte oder auch konzentrierte Schwefelsäure oder Methansulfonsäure. Arbeitet man in nicht-wässrigen Lösemitteln, so ist auch die Anwendung von P₂O₅ oder Molekularsieb denkbar. Man setzt üblicherweise 0,1 bis 20 mol-% Katalysator, bezogen auf I ein; bevorzugt 1 bis 10 mol-%.

Als Reaktionszeit für die erfindungsgemäße Umsetzung sind 10 Minuten bis 24 Stunden sinnvoll, bevorzugt eine bis drei Stunden.

Nach dem Erhitzen arbeitet man üblicherweise auf, indem man zunächst die Säure neutralisiert, beispielsweise mit wässriger Alkalimetallhydroxidlösung oder mit wässriger Alkalimetallcarbonatlösung oder auch mit festen basischen Alkalimetallverbindungen, beispielsweise Alkalimetallhydroxid, Alkalimetallcarbonat oder Alkalmetallbicarbonat.

Anschließend kann man die flüchtigen Bestandteile des Reaktionsgemisches abdestillieren. Dazu ist in der Regel ein Erwärmen auf 40 bis 80°C bei vermindertem Druck, beispielsweise 10 bis 100 mbar, sinnvoll.

In einer bevorzugten Ausführungsform setzt man Aldehyde der allgemeinen Formel I mit 1 bis 1.000 mol-%, bevorzugt 10 bis 500 mol-%, besonders bevorzugt 20 bis 200 mol-% mindestens einer weiteren Carbonylverbindung der Formel II um.

Die als weitere Reaktionspartner eingesetzten Carbonylverbindungen der Formel II trager α-Wasserstoffatome.

Besonders bevorzugt sind Carbonylverbindungen der allgemeinen Formel IIa 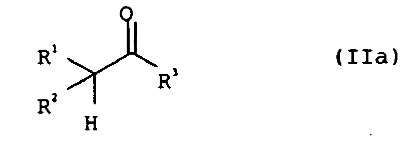 worin R¹ bis R³ die gleiche, bereits zuvor exemplarisch aufgeführte Bedeutung wie in Formel II besitzt.

In einer besonderen Ausführungsform sind die Reste R¹ und R² miteinander unter Bildung eines 4- bis 13-gliedrigen Rings kovalent verbunden. So können dementsprechend R¹ und R² beispielsweise gemeinsam sein: -(CH₂)₄-, -(CH₂)₅-, -(CH₂)₆-, -(CH₂)₇-, -CH(CH₃)-CH₂-CH₂-CH(CH₃)- oder -CH(CH₃)-CH₂-CH₂-CH₂-CH(CH₃)-.

Besonders bevorzugt sind R¹ und R² jeweils Wasserstoff , und R³ ist Methyl.

Für den Fall, dass in Formel I Z einer chemischen Einfachbindung oder einem Rest ohne α-Wasserstoffatomen entspricht, ist die Gegenwart von mindestens einem weiteren Aldehyd der Formel I, in welchem der Rest Z α-Wasserstoffatome enthält, oder mindestens einer weiteren Carbonylverbindung der Formel II zwingend.

Der Überschuss an dem mindestens einen weiteren Aldehyd der Formel I oder der mindestens einen weiteren Carbonylverbindung der Formel II liegt bei mindestens 100 mol-% bezogen auf I.

Ein weiterer Gegenstand der vorliegenden Erfindung ist ein Verfahren zur Herstellung der erfindungsgemäßen Gerbmittel, welches dadurch gekennzeichnet ist, dass man unter Berücksichtigung der zuvor aufgeführten Maßgabe einen oder mehrere Aldehyde der allgemeinen Formel I in Gegenwart mindestens einer weiteren Carbonylverbindung der Formel II in Anwesenheit von Säuren erhitzt.

Bei den erfindungsgemäßen Umsetzungen unter den oben beschriebenen Bedingungen kommt es üblicherweise zur Bildung von schwer trennbaren Gemischen, deren Produkte aus Dimerisierungen, Oligomerisierungen (3 bis 8 Einheiten) und Polymerisation (9 und mehr Einheiten) des Aldehyds der allgemeinen Formel I resultieren, weiterhin aus Aldoladditionen, möglicherweise gefolgt beispielsweise durch Wasserabspaltung (Dehydratisierung), Oxidationen oder auch durch intramolekulare Vernetzung. Bei der Lagerung der erfindungsgemäßen Gerbmittel kann es fernerhin zu lagerungsbedingten Nebenprodukten kommen, beispielsweise durch Wasserabspaltung
(Dehydratisierung), Oxidationen oder auch durch Dimerisierung, Oligomerisierung oder Polymerisation sowie durch Vernetzung.

Es ist möglich, die einzelnen Produkte der erfindungsgemäßen Umsetzungen zu isolieren und als Gerbmittel einzusetzen. Ein bevorzugter Aspekt der vorliegenden Erfindung ist jedoch die Verwendung der nur unvollständig oder gar nicht weiter aufgereinigten Umsetzungsprodukte als Gerbmittel.

Die erfindungsgemäßen Gerbmittel lassen sich zur Vorgerbung, Gerbung und Nachgerbung von Tierhäuten einsetzen. Weitere Gegenstände der vorliegenden Erfindung sind daher die Verwendung der erfindungsgemäßen Gerbmittel zum Vorgerben, Gerben und Nachgerben von Tierhäuten sowie ein Verfahren zum Vorgerben, Gerben oder Nachgerben von Tierhäuten unter Verwendung der erfindungsgemäßen Gerbmittel.

Das erfindungsgemäße Verfahren zum Vorgerben, Gerben oder Nachgerben von Tierhäuten, im Folgenden auch erfindungsgemäßes Gerbverfahren genannt, geht aus von nach konventionellen Methoden vorbehandelten Häuten von Tieren wie beispielsweise Rindern, Schweinen, Ziegen oder Hirschen. Dabei ist es für das erfindungsgemäßen Gerbverfahren nicht wesentlich, ob die Tiere beispielsweise durch Schlachten getötet wurden oder aber an natürlichen Ursachen verendet sind. Zu den konventionellen Methoden der Vorbehandlung gehören das beispielsweise das Äschern, Entkälken,
Beizen und Pickeln sowie mechanische Arbeitschritte, beispielsweise zur Entfleischung der Häute.

Das erfindungsgemäße Gerbverfahren übt man im Allgemeinen so aus, dass man ein oder mehrere erfindungsgemäße Gerbmittel in einer Portion oder in mehreren Portionen unmittelbar vor oder aber während des Gerbungsschrittes zusetzt. Das erfindungsgemäße Gerbverfahren wird vorzugsweise bei einem pH-Wert von 2,5 bis 4 durchgeführt, wobei man häufig beobachtet, dass der pH-Wert während der Durchführung des erfindungsgemäßen Gerbverfahrens um etwa 0,3 bis drei Einheiten ansteigt. Man kann den pH-Wert auch durch Zugabe abstumpfender Mittel um etwa 0,3 bis drei Einheiten erhöhen.

Das erfindungsgemäße Gerbverfahren führt man im Allgemeinen bei Temperaturen von 10 bis 45°, bevorzugt bei 20 bis 30°C durch. Bewährt hat sich eine Dauer von 10 Minuten bis 12 Stunden, bevorzugt sind eine bis drei Stunden. Das erfindungsgemäße Gerbverfahren kann man in beliebigen gerbereiüblichen Gefäßen durchführen, beispielsweise durch Walken in Fässern.

In einer Variante des erfindungsgemäßen Gerbverfahrens setzt man das oder die erfindungsgemäßen Gerbmittel zusammen mit einem oder mehreren herkömmlichen Gerbstoffen ein, beispielsweise mit Chromgerbstoffen oder anderen mineralischen Gerbstoffen, Syntanen, Polymergerbstoffen oder vegetabilen Gerbstoffen, wie sie beispielsweise beschrieben sind in Ullmann's Encyclopedia of Industrial Chemistry, Band A15, Seite 259 bis 282 und insbesondere Seite 268 ff., 5. Auflage, (1990), Verlag Chemie Weinheim. Das Gewichtsverhältnis erfindungsgemäßes Gerbmittel : herkömmlicher Gerbstoff bzw. Summe der herkömmlichen Gerbstoffe beträgt zweckmäßig von 0,01 : 1 bis 100 : 1. In einer vorteilhaften Variante des erfindungsgemäßen Verfahrens setzt man nur wenige ppm der herkömmlichen Gerbmittel den erfindungsgemäßen Gerbmitteln zu. Besonders vorteilhaft ist es jedoch, auf die Beimischung herkömmlicher Gerbstoffe ganz zu verzichten.

In einer Variante des erfindungsgemäßen Gerbverfahrens setzt man ein oder mehrere erfindungsgemäße Gerbmittel in einer Portion oder in mehreren Portionen vor oder während des Vorgerbens zu, in einer besonderen Variante bereits im Pickel.

In einer weiteren Variante des erfindungsgemäßen Gerbverfahrens setzt man ein oder mehrere erfindungsgemäße Gerbmittel in einer Portion oder in mehreren Portionen vor oder während eines oder mehrerer Nachgerbungsschritte zu. Diese Variante wird im Folgenden als erfindungsgemäßes Nachgerbverfahren bezeichnet. Das erfindungsgemäße Nachgerbverfahren kann man unter ansonsten üblichen Bedingungen durchführen. Man wählt zweckmäßig einen oder mehrere, d.h. 2 bis 6 Einwirkschritte und kann zwischen den Einwirkschritten mit Wasser spülen. Die Temperatur bei den einzelnen Einwirkschritten beträgt jeweils von 5 bis 60°C, bevorzugt 20 bis 45°C. Man setzt zweckmäßig weitere, während der Nachgerbung üblicherweise verwendete Mittel ein, beispielsweise Fettlicker, Polymergerbstoffe und Fettungsmittel auf Acrylat- und/oder Methacrylatbasis, Nachgerbstoffe auf Basis von Harz- und Vegetabilgerbstoffen, Füllstoffe, Lederfarbstoffe oder Emulgatoren.

Ein weiterer Aspekt der vorliegenden Erfindung sind Leder, hergestellt unter Verwendung des erfindungsgemäßen Gerbmittels oder nach dem erfindungsgemäßen Gerbverfahren und/oder Nachgerbverfahren. Die erfindungsgemäßen Leder zeichnen sich durch eine insgesamt vorteilhafte Qualität aus, beispielsweise glatten Narben, homogenere Gerbung über den Querschnitt, verbesserte Reißfestigkeit und Fülle sowie geringere Neigung zum Verfärben, insbesondere zum Vergilben.

In einer speziellen Ausführungsform des erfindungsgemäßen Gerbverfahrens setzt man die erfindungsgemäßen Gerbmittel in Form pulverförmiger Wirkstoffe ein. Ein weiterer Gegenstand des erfindungsgemäßen Verfahrens betrifft daher pulverförmige Wirkstoffe, enthaltend
10 bis 100 Gew.-%, bevorzugt 40 bis 90 Gew.-% eines oder mehrerer erfindungsgemäßer Gerbmittel und
0 bis 90 Gew.-%, bevorzugt 10 bis 60 Gew.-% eines oder mehrerer Zuschlagstoffe.

Die Zuschlagstoffe sind in der Regel feste partikuläre Stoffe. Bevorzugt werden sie gewählt aus Stärke, Siliziumdioxid, beispielsweise in der Form von Kieselgel, insbesondere sphäroidale Kieselgele, Schichtsilikate, Aluminiumoxid sowie Mischoxiden von Silizium und Aluminium.

Als Zuschlagstoffe sind weiterhin ein oder mehrere herkömmliche Gerbstoffe oder Nachgerbstoffe, insbesondere Harzgerbstoffe, beispielsweise der unter dem Namen Relugan® D, Tamol® M und Basyntan® DLX von der BASF Aktiengesellschaft vertriebene Harzgerbstoff. Auch Ligninsulfonate sind geeignete Zuschlagstoffe.

Die erfindungsgemäßen pulverförmigen Wirkstoffe sind weiterhin charakterisiert dadurch, dass sie aus feinen Partikeln mit einem mittleren Partikeldurchmesser von 100 nm bis 0,1 mm bestehen. Die Partikeldurchmesser folgen dabei einer Partikeldurchmesserverteilung, die eng oder breit sein kann. Auch bimodale Partikelgrößenverteilungen sind denkbar. Die Partikel selbst können irregulär oder sphärischer Form sein, wobei sphärische Partikelformen bevorzugt sind. Die erfindungsgemäßen pulverförmigen Wirkstoffe lassen sich im erfindungsgemäßen Gerbverfahren bzw. Nachgerbverfahren unter besonders hygienischen Verhältnissen dosieren.

Ein weiterer Gegenstand der vorliegenden Erfindung betrifft ein Verfahren zur Herstellung der erfindungsgemäßen pulverförmigen Wirkstoffe. Das erfindungsgemäße Verfahren geht aus von in Lösung, in Suspension oder Emulsion oder aber von isoliert vorliegenden erfindungsgemäßen Gerbmitteln. Besonders bevorzugt geht man von Reaktionslösungen aus, wie sie im erfindungsgemäßen Herstellungsverfahren bei der Herstellung der erfindungsgemäßen Gerbmittel anfallen.

Es hat sich bewährt, die Reaktionslösungen zunächst bis zu einem Restlösemittelgehalt von 50 Gew.-% oder weniger aufzukonzentrieren.

Erfindungsgemäß versprüht man die anfallenden flüssigen, festen oder öligen aufkonzentrierten Reaktionslösungen in einem Sprühtrockner, bevorzugt in einem Sprühturm. Sprühtrockner sind dem Fachmann bekannt und beispielsweise beschrieben in Vauck/Müller, Grundoperationen chemischer Verfahrenstechnik, VCH Weinheim, 1988, 7. Auflage, S. 638-740 und S. 765-766, sowie in der darin zitierten Literatur.

Ein weiterer Aspekt der vorliegenden Erfindung betrifft die Verwendung der erfindungsgemäßen Gerbmittel als Konservierungsmittel sowie Konservierungsmittel, enthaltend die erfindungsgemäßen Gerbmittel sowie übliche Zusätze. Die erfindungsgemäßen Gerbmittel bzw. Konservierungsmittel eignen sich zur Konservierung von Oberflächen und Erzeugnissen, beispielsweise kosmetischen Erzeugnissen.

Die Erfindung wird durch die nachfolgenden Beispiele näher erläutert, ohne dass jedoch hierdurch eine Einengung des Schutzumfangs impliziert werden soll.
1. Herstellung der erfindungsgemäßen Gerbmittel
   1.1 Herstellung aus Glutaraldehyd und Aceton
   Ein 2-Liter-Dreihalskolben mit Kühler, Rührer und Thermometer wurde mit 900 g einer 50 Gew.-% wässrigen Lösung von Glutaraldehyd (4,5 mol), 420 g (7,2 mol) Aceton und 18 ml einer 50 Gew.-% Schwefelsäure beschickt. Man rührte 3 Stunden bei 70-73 °C und kühlte anschließend ab. Anschließend wurde der Kühler gegen eine Destillationsbrücke ausgetauscht. Aceton wurde bis zu einer Sumpftemperatur von 78°C binnen 60 Minuten abdestilliert. Das Gemisch wurde mit 14 ml 50 Gew.-% wässriger Natronlauge auf pH-Wert von 4,5-5,5 gestellt. Anschließend wurden noch 20 Minuten bei 60-65°C im Vakuum (10mbar) flüchtige Bestandteile abdestilliert. Man erhielt 830 g einer bernsteinfarbenen Flüssigkeit. Die Viskosität wurde zu 45 mPa.s (20°C) bestimmt. Die GPC-Analyse zeigte eine breite Molmassenverteilung (Q = 9,6) mit Mₙ = 450 g.
   1.2 Herstellung aus Glutaraldehyd und Butanon
   Ein 2-Liter-Dreihalskolbenmit Kühler, Rührer und Thermometer wurde mit 900 g 50 Gew.-% wässriger Lösung von Glutaraldehyd(4,5 mol), 520 g (7,2 mol) Butanon und 20 ml einer 50 Gew.-% Schwefelsäure beschickt. Man rührte 3 Stunden bei 80°C und kühlte anschließend ab. Unter Rühren setzte man bei 50°C 16 ml einer 50 Gew.-% wässrigen Natronlauge zu. Anschließend wurde der Kühler gegen eine Destillationsbrücke ausgetauscht. Danach wurde das Gemisch eine Stunde bei 10 mbar und 60 bis 65°C erwärmt und flüchtige Bestandteile abdestilliert. Man erhielt 860 g einer braunen Flüssigkeit. Die Viskosität wurde zu 35 m·Pas (20°C) bestimmt. Die GPC-Analyse zeigte eine breite Molmassenverteilung (Q = 11,3) mit Mₙ = 420 g.
   1.3 Herstellung aus Glutaraldehyd und Acetaldehyd
   Ein 2-Liter-Dreihalskolbenmit Kühler, Rührer und Thermometer wurde mit 800 g 50 Ges.-% wässriger Lösung von Glutaraldehyd (4 mol), 520 g einer 35 Ges.-% wässrige Lösung Acetaldehyd (4, 1 mol) und 20 ml einer 50%igen Schwefelsäure beschickt. Man rührte 3 Stunden bei 80°C und kühlte anschließend ab. Unter Rühren setzte man bei 50°C 16 ml einer 50 Gew.-% wässrigen Natronlauge zu. Anschließend wurde das Gemisch 1 h bei 10 mbar auf 60-65°C erwärmt und flüchtige Bestandteile abdestilliert. Man erhielt 840 g einer bernsteinfarbenen Flüssigkeit. Die Viskosität der betrug 22 mPa·s (20°C) bestimmt. Die GPC-Analyse zeigte eine breite Molmassenverteilung (Q = 7,3) mit Mₙ = 360 g.
   1.4 Herstellung aus Glutaraldehyd und Benzaldehyd
   Ein 2-Liter-Dreihalskolbenmit Kühler, Rührer und Thermometer wurde mit 800 g 50 Gew.-% wässrigerGlutaraldehyd-Lösung (4 mol), 350 g Benzaldehyd (3,3 mol) und 18 ml einer 50%igen Schwefelsäure beschickt. Man rührte kräftig über einen Zeitraum von 3 Stunden bei 80°C und kühlte anschließend ab. Unter Rühren setzte man bei 50°C 14 ml einer 50 Gew. -% wässrigen Natronlauge zu. Man trennte die wässrige Phase im Scheidetrichter ab. Anschließend wurde das Gemisch 1 h bei 10 mbar auf 60-65°C erwärmt und flüchtige Bestandteile abdestilliert. Man erhielt 640 g einer bernsteinfarbenen Flüssigkeit. Die Viskosität betrug 38 mPa·s (20°C). Die GPC-Analyse zeigte eine breite Molmassenverteilung (Q = 9,6) mit Mₙ = 680 g.
   1.5 Herstellung aus Glutaraldehyd und Benzaldehyd
   Ein 2-Liter-Dreihalskolbenmit Kühler, Rührer und Thermometer wurde mit 800 g 50 Gew.-% wässrigerGlutaraldehyd-Lösung (4 mol), 350 g Benzaldehyd (3,3 mol) und 18 ml einer 50%igen Schwefelsäure beschickt. Man rührte kräftig über einen Zeitraum von 3 Stunden bei 80°C und kühlte anschließend ab. Unter Rühren setzte man bei 50°C 14 ml einer 50 Gew.-% wässrigen Natronlauge zu. Man trennte die wässrige Phase im Scheidetrichter ab. Anschließend wurde das Gemisch 1 h bei 10 mbar auf 60-65°C erwärmt und flüchtige Bestandteile abdestilliert. Man erhielt 640 g einer bernsteinfarbenen Flüssigkeit. Die Viskosität betrug 38 mPa·s (20°C). Die GPC-Analyse zeigte eine breite Molmassenverteilung (Q = 9,6) mit Mₙ = 680 g.
   1.6 Herstellung eines erfindungsgemäßen pulverförmigen Wirkstoffs
   300 g des in Beispiel 1.1 hergestellten Gerbmittels wurden bei 50°C mit 200 ml einer 50 Gew.-% wässrigen Stärkelösung versetzt (Maltodextrin plus®) und innig durchmischt. Anschließend wurde die Lösung durch Sprühtrocknung in einem Sprühturm der Fa. APV; Typ Lab S1 in ein beigefarbenes Pulver überführt (Ausbeute 280 g). Der Sprühturm wurde in der folgenden Einstellung betrieben:
   Eintrittstemperatur 300°C / Austrittstemperatur 90°C Durchsatz: 7,5 kg / h . Elektrolufterhitzer: 9 kW Druckluft-Verbrauch: 7,2 m³ / h Druck (Druckluft): 4 bar
2. Gerbversuche 2.1 bis 2.5 und Vergleichsbeispiele, Chrom-freie Gerbung
   Etwa 500 g bei pH 3, 0-3, 2 gepickelte Rindsblöße wurden bei 25°C in einem 10 1 Fass mit 350 ml Wasser und, bezogen auf die Pickelblöße, jeweils 3 Gew. -% der Produkte gemäß Beispiel 1.1 bis 1. versetzt. Nach einer Walkzeit von 45 Minuten wurde mit 0,5 % Magnesiumoxid (Neutrigan® MO, BASF Aktiengesellschaft) binnen 6 Stunden der pH auf 4, 9-5, 1 gestellt. Die Flotte wurde abgelassen und die Haut mit 300 ml Wasser gewaschen.

Die Ergebnisse sind in Tabelle 1 aufgeführt.

Zum Vergleich wurden gepickelte Rindsblößen mit
- 3 Gew. -% Glutaraldehyd (50 Gew. -% wässrige Lösung, Relugan® GT50 der BASF Aktiengesellschaft, V 2.7)
- 4 Gew.-% Glutaraldehyd (24 Gew.-% wässrige Lösung, Relugan® GT 24 der BASF Aktiengesellschaft, V 2.8)
- 5 Gew.-% einer 25 Gew.-% wässrigen Formaldehydlösung versetzt (V 2.9) und jeweils analog zu oben weiter gearbeitet.

Es wurden die Schrumpftemperaturen gemäß der Vorschrift nach DIN 53 336 (1977) bestimmt, wobei die DIN-Vorschrift wie folgt modifiziert wurde:
Punkt 4.1: Die Probestücke hatten die Abmessungen 3 cm·1 cm, die Dicke wurde nicht bestimmt;
Punkt 4.2: es wurde nur eine anstatt 2 Proben pro Ledermuster geprüft.
Punkt 6: entfiel.
Punkt 7: Die Trocknung im Vakuum-Exsikkator entfiel.
Punkt 8: Bei Rückgang des Zeigers wurde die Schrumpfungstemperatur abgelesen.

**Tabelle 1: Gerbung mit erfindungsgemäßen Gerbmitteln sowie vergleichsversuche; Analyse der erfindungsgemäßen Leder**

| Gerbung | | | Analyse der Leder | |
|---|---|---|---|---|
| Nr. | Aldehyd I / Carbonylverbindung II | Dosierung * [Gew.-%] | Schrumpf temperatur [°C] | Vergilbung [Benotung 1-5] |
| 2.1 | Glutaraldehyd/Aceton | 3 | 77,5 | 2 |
| 2.2 | Glutaraldehyd/Butanon | 3 | 75 | 3 |
| 2.3 | Glutaraldehyd/Acetaldehyd | 3 | 74 | 3,5 |
| 2.4 | Glutaraldehyd/Benzaldehyd | 3 | 76 | 3,5 |
| 2.5 | Glutaraldehyd/Aceton/ Stärke | 3 | 75 | 2 |
| V2.7 | Glutaraldehyd 50 Gew.-% | 3 | 79 | 4 |
| V2.8 | Glutaraldehyd 24 Gew.-% | 4 | 75 | 3,5 |
| V 2.9 | Formalin 25 Gew.-% | 5 | 73 | 2,5 |

| | | | | |
|---|---|---|---|---|
| * bezogen auf die Pickelblöße | | | | |

Die Benotung erfolgt wie in der Schule; 1: sehr gut, 5: mangelhaft.

Es wurde außerdem die Konzentration von Glutaraldehyd in der Gasphase über den erfindungsgemäßen Gerbmitteln 1.1 bis 1.6 bei 25°C und 40°C per GC/MS bestimmt. Weiterhin wurde jeweils 20 Minuten nach Dosierung der Gerbmittel die Konzentration von Glutaraldehyd über den Gerbflotten aus den Beispielen 2.1 bis 2.6 sowie V 2.7 und V 2.8 bei 25°C gemäß der oben stehenden Beispiele bestimmt:
Die Ergebnisse sind in Tabelle 2 zusammengefasst.

**Tabelle 2: Untersuchungen zur Bestimmung der Konzentration des freien Glutaraldehyds in der Luft**

| Gerbmittel Nr. | Konzentration Glutaraldehyd bei 25°C [ppm] | Konzentration Glutaraldehyd bei 40°C [ppm] | Gerbflotte aus Beispiel | Konzentration Glutaraldehyd bei 25°C [ppm] |
|---|---|---|---|---|
| 1.1 | 5 | 8 | 2.1 | 2 |
| 1.2 | 3 | 7 | 2.2 | 1 |
| 1.3 | 3 | 7 | 2.3 | 1 |
| 1.4 | 5 | 9 | 2.4 | 2 |
| 1.5 | 2 | 5 | 2.5 | 2 |
| 1.6 | 2 | 4 | 2.6 | nicht nachweisbar |
| Glutaraldehyd 50 Gew.-% | 12 | 34 | V 2.7 | 8 |
| Glutaraldehyd 24 Gew.-% | 16 | 56 | V 2.8 | 15 |

## Patentansprüche

1. Gerbmittel, herstellbar durch Umsetzen mindestens eines Aldehyds der allgemeinen Formel I, 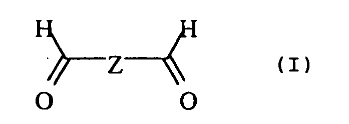 mit mindestens einem weiteren identischen oder verschiedenen Aldehyd der Formel I,
wobei die Variablen wie folgt definiert sind:
Z eine chemische Einfachbindung, gegebenenfallssubstituiertes C₁-C₁₂-Alkylen, gegebenenfalls substituiertes C₅-C₁₂-Cycloalkylen oder gegebenenfalls substituiertes C₆-C₁₄-Arylen,
wobei man die Umsetzung in Anwesenheit eines sauren Katalysators und in Gegenwart mindestens einer weiteren Carbonylverbindung der Formel II 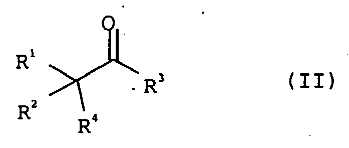 durchführt,
wobei
R¹ bis R⁴ unabhängig voneinander Wasserstoff, gegebenenfalls substituiertes C₁-C₁₂- Alkyl, gegebenenfalls substituiertes C₃-C₁₂-Cycloalkyl, gegebenenfalls substituiertes C₇-C₁₃-Aralkyl oder gegebenenfalls substituiertes C₆-C₁₄-Aryl bedeuten,
mit der Maßgabe, dass für den Fall, dass Z einer chemischen Einfachbindung oder einem Rest ohne α-Wasserstoffatomen entspricht, mindestenseinweiterer Aldehydder Formel I, inwelchem der Rest Z α-Wasserstoffatome enthält, odermindestenseineweitere Carbonylverbindung der Formel II, die α-H-Atome trägt, vorhanden ist.

2. Gerbmittel nach Anspruch 1, **dadurch gekennzeichnet, dass** man den Aldehyd der Formel I in Gegenwart von 1 bis 1.000 mol-% mindestens einer weiteren Carbonylverbindung der Formel II erhitzt.

3. Gerbmittel nach Anspruch 1 oder 2, **dadurch gekennzeichnet, dass** die weitere Carbonylverbindung der Formel II α-Wasserstoffatome aufweist.

4. Gerbmittel nach Anspruch 1 bis 3, **dadurch gekennzeichnet, dass** die weitere Carbonylverbindung der allgemeinen Formel IIa 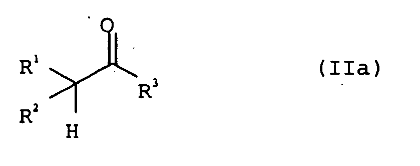 entspricht,
wobei
R¹ bis R³ die gleiche Bedeutung wie in Formel II von Anspruch 1 besitzt.

5. Gerbmittel nach einem oder mehreren der Ansprüche 1 bis 4, **dadurch gekennzeichnet, dass** R³ in Formel II Methyl ist.

6. Gerbmittel nach einem oder mehreren der Ansprüche 1 bis 5, **dadurch gekennzeichnet, dass** man im Anschluss an die Umsetzung die Säure neutralisiert und flüchtige Bestandteile abdestilliert.

7. Verfahren zur Herstellung von Gerbmitteln nach einem oder mehreren der Ansprüche 1 bis 6, **dadurch gekennzeichnet, dass** man unter Berücksichtigung der Maßgabe in Anspruch 1 einen oder mehrere Aldehyde der allgemeinen Formel I in Gegenwart mindestens einer weiteren Carbonylverbindung der Formel II in Anwesenheit von Säuren erhitzt.

8. Verwendung von Gerbmitteln nach einem oder mehreren der Ansprüche 1 bis 6 zum Vorgerben, Gerben oder Nachgerben von Tierhäuten.

9. Verfahren zum Vorgerben, Gerben oder Nachgerben von Tierhäuten, **dadurch gekennzeichnet, dass** man Gerbmittel nach einem oder mehreren der Ansprüche 1 bis 6 einsetzt.

10. Verfahren nach Anspruch 9, **dadurch gekennzeichnet, dass** man das oder die Gerbmittel als pulverförmigen Wirkstoff einsetzt.

11. Pulverförmiger Wirkstoff, enthaltend
10 bis 100 Ges.-% eines oder mehrerer Gerbmittel nach einem oder mehreren der Ansprüche 1 bis 6 und
0 bis 90 Gew.-% eines oder mehrerer Zuschlagstoffe.

12. Pulverförmiger Wirkstoff nach Anspruch 11, in dem der oder die Zuschlagstoffe gewählt werden aus Stärke, Siliziumdioxid, Aluminiumoxid sowie Mischoxiden von Silizium und Aluminium.

13. Verfahren zur Herstellung von pulverförmigen Wirkstoffen nach Anspruch 11 oder 12, **dadurch gekennzeichnet, dass** man sie durch Sprühtrocknung gewinnt.

14. Leder, hergestellt unter Verwendung eines Gerbmittels gemäß Anspruch 8 oder nach einem Verfahren nach Anspruch 9.

15. Verwendung von Gerbmitteln nach einem oder mehreren der Ansprüche 1 bis 6 als Konservierungsmittel.

16. Verwendung von pulverförmigen wirkstoffen nach Anspruch 11 oder 12 als Konservierungsmittel.

## Claims

1. A tanning agent which can be prepared by reacting at least one aldehyde of the formula I, 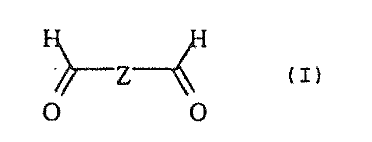 with at least one further identical or different aldehyde of the formula I,
where the variables are defined as follows:
z is a single chemical bond, unsubstituted or substituted C₁-C₁₂-alkylene, unsubstituted or substituted C₅-C₁₂-cycloalkylene or unsubstituted or substituted C₆-C₁₄-arylene,
the reaction being carried out in the presence of an acidic catalyst and in the presence of at least one further carbonyl compound of the formula II 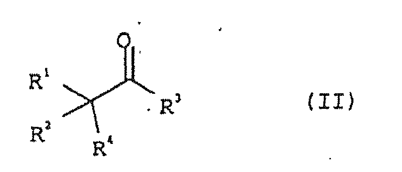 where
R¹ to R⁴, independently of one another, are hydrogen, unsubstituted or substituted C₁-C₁₂-alkyl, unsubstituted or substituted C₃-C₁₂-cycloalkyl, unsubstituted or substituted C₇-C₁₃-aralkyl or unsubstituted or substituted C₆-C₁₄-aryl ,
with the proviso that, where Z is a single chemical bond or a radical without α-hydrogen atoms, at least one further aldehyde of the formula I, in which Z comprises α-hydrogen atoms, or at least one further carbonyl compound of the formula II which carries α-H atoms is present.

2. The tanning agent according to claim 1, wherein the aldehyde of the formula I is heated in the presence of from 1 to 1 000 mol % of at least one further carbonyl compound of the formula II.

3. The tanning agent according to claim 1 or 2, wherein the further carbonyl compound of the formula II has α-hydrogen atoms.

4. The tanning agent according to any of claims 1 to 3, wherein the further carbonyl compound corresponds to the formula IIa 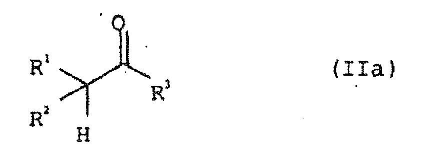 where
R¹ to R³ are as defined in formula II of claim 1.

5. The tanning agent according to one or more of claims 1 to 4, wherein R³ in formula II is methyl.

6. The tanning agent according to one or more of claims 1 to 5, wherein, after the reaction, the acid is neutralized and volatile components are distilled off.

7. The process for the preparation of a tanning agent according to one or more of claims 1 to 6, wherein, taking into account the proviso stated in claim 1, one or more aldehydes of the formula I are heated in the presence of acids and in the presence of at least one further carbonyl compound of the formula II.

8. The use of a tanning agent according to one or more of claims 1 to 6 for pretanning, tanning or retanning animal hides.

9. A process for pretanning, tanning or retanning animal hides, wherein a tanning agent according to one or more of claims 1 to 6 is used.

10. The process according to claim 9, wherein the tanning agent or tanning agents is or are used in the form of a pulverulent active ingredient.

11. A pulverulent active ingredient comprising
from 10 to 100% by weight of one or more tanning agents according to one or more of claims 1 to 6 and
from 0 to 90% by weight of one or more additives.

12. The pulverulent active ingredient according to claim 11, in which the additive or additives is or are selected from starch, silica, alumina and mixed oxides of silicon and aluminum.

13. A process for the preparation of a pulverulent active ingredient according to claim 11 or 12, wherein it is obtained by spray-drying.

14. A leather produced using a tanning agent according to claim 8 or by a process according to claim 9.

15. The use of a tanning agent according to one or more of claims 1 to 6 as a preservative.

16. The use of a pulverulent active ingredient according to claim 11 or 12 as preservative.

## Revendications

1. Agent de tannage, pouvant être préparé par mise en réaction d'au moins un aldéhyde de formule générale I, 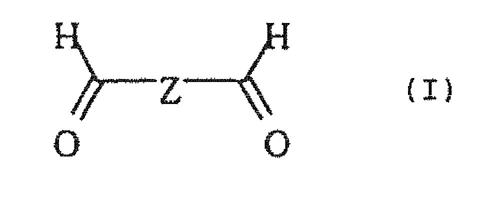 avec au moins un autre aldéhyde, identique ou différent, de formule I,
les variables étant définies comme suit :
Z représente une liaison chimique simple, un groupe alkylène en C₁-C₁₂ éventuellement substitué, cycloalkylène en C₅-C₁₂ éventuellement substitué ou arylène en C₆-C₁₄ éventuellement substitué,
en effectuant la réaction en présence d'un catalyseur acide et en présence d'au moins un autre composé carbonyle de formule II 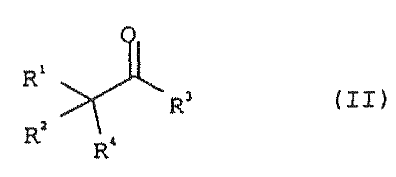 dans laquelle
R¹ à R⁴ représentent, indépendamment les uns des autres, un atome d'hydrogène, un groupe alkyle en C₁-C₁₂ éventuellement substitué, cycloalkyle en C₃-C₁₂ éventuellement substitué, aralkyle en C₇-C₁₃ éventuellement substitué ou aryle en C₆-C₁₄ éventuellement substitué,
avec la condition que lorsque Z correspond à une liaison chimique simple ou à un radical sans atomes d'hydrogène α, est présent au moins un autre aldéhyde de formule I, dans lequel le radical Z contient des atomes d'hydrogène α, ou au moins un autre composé carbonyle de formule II qui porte des atomes d'hydrogène α.

2. Agent de tannage selon la revendication 1,
**caractérisé en ce que** l'aldéhyde de formule I est chauffé en présence de 1 à 1 000 % en moles d'au moins un autre composé carbonyle de formule II.

3. Agent de tannage selon la revendication 1 ou 2, **caractérisé en ce que** l'autre composé carbonyle de formule II comporte des atomes d'hydrogène α.

4. Agent de tannage selon l'une quelconque des revendications 1 à 3, **caractérisé en ce que** l'autre composé carbonyle correspond à la formule générale IIa 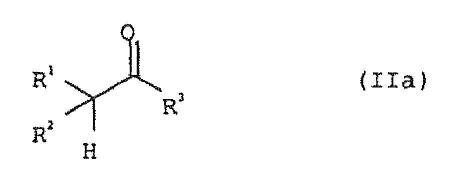 dans laquelle
R¹ à R³ ont les mêmes significations que dans la formule II de la revendication 1.

5. Agent de tannage selon une ou plusieurs des revendications 1 à 4, **caractérisé en ce que** R³ dans la formule II est le groupe méthyle.

6. Agent de tannage selon une ou plusieurs des revendications 1 à 5, **caractérisé en ce qu'**à la suite de la réaction on neutralise l'acide et on élimine par distillation les composants volatils.

7. Procédé pour la préparation d'agents de tannage selon une ou plusieurs des revendications 1 à 6, **caractérisée en ce qu'**en tenant compte de la condition énoncée dans la revendication 1 on chauffe en présence d'acides un ou plusieurs aldéhydes de formule générale I en présence d'au moins un autre composé carbonyle de formule II.

8. Utilisation d'agents de tannage selon une ou plusieurs des revendications 1 à 6 pour le pré-tannage, le tannage ou le post-tannage de peaux animales.

9. Procédé pour le pré-tannage, le tannage ou le post-tannage de peaux animales, **caractérisé en ce qu'**on utilise des agents de tannage selon une ou plusieurs des revendications 1 à 6.

10. Procédé selon la revendication 9, **caractérisé en ce qu'**on utilise l'agent ou les agents de tannage sous forme de substance active pulvérulente.

11. Substance active pulvérulente, contenant
10 à 100 % en poids d'un ou plusieurs agents de tannage selon une ou plusieurs des revendications 1 à 6 et
0 à 90 % en poids d'un ou plusieurs additifs.

12. Substance active pulvérulente selon la revendication 11, dans laquelle l'additif ou les additifs sont choisis parmi l'amidon, le dioxyde de silicium, l'oxyde d'aluminium ainsi que des oxydes mixtes de silicium et aluminium.

13. Procédé pour la préparation de substances actives pulvérulentes selon la revendication 11 ou 12, **caractérisé en ce qu'**on les obtient par séchage par atomisation.

14. Cuir, produit avec utilisation d'un agent de tannage selon la revendication 8 ou selon un procédé conforme à la revendication 9.

15. Utilisation d'agents de tannage selon une ou plusieurs des revendications 1 à 6, en tant que conservateur.

16. Utilisation de substances actives selon la revendication 11 ou 12, en tant que conservateur.
